(19) **Europäisches Patentamt**

**European Patent Office**

**Office européen des brevets**

(11) **EP 1 286 769 B1**

(12) **EUROPEAN PATENT SPECIFICATION**

(45) Date of publication and mention
of the grant of the patent:
**29.09.2004 Bulletin 2004/40**

(51) Int Cl.[7]: **C07C 2/58**, B01J 35/10,
B01J 29/06

(21) Application number: **01943424.0**

(22) Date of filing: **21.05.2001**

(86) International application number:
**PCT/EP2001/005845**

(87) International publication number:
**WO 2001/091901 (06.12.2001 Gazette 2001/49)**

(54) **USE OF A CATALYST FOR THE ALKYLATION OF HYDROCARBONS**

VERWENDUNG EINES KATALYSATORS ZUR ALKYLIERUNG VON KOHLENWASSERSTOFFEN

UTILISATION D'UN CATALYSEUR POUR L'ALKYLATION D'HYDROCARBURES

(84) Designated Contracting States:
**AT BE CH CY DE DK ES FI FR GB GR IE IT LI LU
MC NL PT SE TR**

(30) Priority: **30.05.2000 EP 00201917**

(43) Date of publication of application:
**05.03.2003 Bulletin 2003/10**

(73) Proprietor: **Akzo Nobel N.V.
6824 BM Arnhem (NL)**

(72) Inventors:
 • **VAN BROEKHOVEN, Emanuel, Hermanus
 NL-1141 DN Monnickendam (NL)**
 • **MAS CABR , Francisco, René
 NL-1183 NE Amstelveen (NL)**

(74) Representative: **Schalkwijk, Pieter Cornelis et al
Akzo Nobel N.V.
Intellectual Property Department
P.O. Box 9300
6800 SB Arnhem (NL)**

(56) References cited:
**EP-A- 0 216 938          EP-A- 0 389 041
WO-A-98/23560          WO-A-98/42805**

Remarks:
The file contains technical information submitted
after the application was filed and not included in this
specification

**Description**

The present invention relates to the use of a catalyst for the alkylation of hydrocarbons.

Within the framework of the present invention, the term alkylation refers to the reaction of a hydrocarbon, such as an aromatic or a saturated hydrocarbon, with an olefin. Without limiting the scope of the invention we will further illustrate the invention by discussing the alkylation of saturated hydrocarbons, in general branched saturated hydrocarbons, with an olefin to give highly branched saturated hydrocarbons with a higher molecular weight.

This reaction is of interest because it makes it possible to obtain, through the alkylation of isobutane with an olefin containing 2-6 carbon atoms, an alkylate which has a high octane number and which boils in the gasoline range. Unlike gasoline obtained by cracking heavier petroleum fractions such as vacuum gas oil and atmospheric residue, gasoline obtained by alkylation is essentially free of contaminants such as sulphur and nitrogen and so has clean burning characteristics. Its high anti-knock properties, represented by the high octane number, lessen the need to add environmentally harmful anti-knock compounds such as lead. Also, unlike gasoline obtained by reforming naphtha or by cracking heavier petroleum fractions, alkylate contains few if any aromatics or olefins, which, environmentally speaking, is a further advantage.

The alkylation reaction is acid-catalyzed. At present, in commercial alkylation equipment use is made of liquid acid catalysts such as sulphuric acid and hydrogen fluoride. The use of such catalysts is attended with a wide range of problems. For instance, sulphuric acid and hydrogen fluoride are highly corrosive, so that the equipment used has to meet high quality requirements. Since the presence of highly corrosive materials in the resulting fuel is objectionable, the remaining acid has to be removed from the alkylate. Also, because of the phase separations which have to be carried out, the process is complicated and thus expensive. Besides, there is always the risk that toxic substances such as hydrogen fluoride will be emitted.

A newer development in this field is the use of solid acid catalysts, such as zeolite-containing catalysts. Thus WO 9823560 describes the use of a catalyst containing a zeolite, such as a Y zeolite, and a hydrogenation function, such as a Group VIII noble metal, e.g., platinum or palladium, and, optionally, a matrix material, such as alumina, in the alkylation of saturated hydrocarbons. Though the performance of this catalyst is satisfactory, there is still a need for further increase of catalytic activity, selectivity, and stability of these catalysts.

It has now surprisingly been found that the performance can be further improved by choosing the catalyst features such that (a) the ratio between (i) the volume in catalyst pores with a diameter in the range of 40 - 8000 nm and (ii) the specific length of the catalyst particles is in the range of 0.01 - 0.90 ml/(g*mm), (b) the total pore volume of the catalyst is at least 0.20 ml/g and (c) the volume in the catalyst pores with a diameter in the range of 40-8000 nm is below 0.30 ml/g.

The invention will be explained in more detail below.

The present invention pertains to the use in the alkylation of hydrocarbons of a catalyst particle comprising a hydrogenation function and a zeolite, wherein the ratio between (i) the volume in catalyst pores with a diameter in the range of 40 - 8000 nm and (ii) the specific length of the catalyst particles is in the range of 0.01 - 0.90 ml/(g*mm) and wherein the catalyst has a total pore volume of at least 0.20 ml/g and the volume in the catalyst pores with a diameter in the range of 40-8000 nm is below 0.30 ml/g.

In the following the pores having a diameter in the range of 40 - 8000 nm will be denoted "macropores" and the pore volume in these pores will be designated as "macropore volume".

The specific length of the catalyst particle is defined as the ratio between the geometric volume and the geometric surface of the solid part of this catalyst particle. The determination of the geometric volume and the geometric surface is known to the person skilled in the art and can be carried out, e.g., as described in DE 2354558. It is noted that the specific length is different from the diameter of the catalyst particle. E.g., for a cylindrical catalyst particle the particle diameter is four to six times higher (depending on the diameter and the length of the particle) than the specific length. Further, the diameter of, e.g., a sphere is six times higher than the specific length.

It is noted that EP 216 938 discloses the use of such catalysts in hydrotreating, but not in alkylation reactions.

As stated above, it is essential to the catalyst used in the present invention that it has a ratio between macropore volume and specific length in the range of 0.01 - 0.90 ml/(g*mm). As further stated above, it is essential to the catalyst used in the present invention that it has a total pore volume of at least 0.20 ml/g and that the macropore volume is below 0.30 ml/g

As is shown in the (comparative) examples below, the catalyst shows a significantly poorer performance in the alkylation of hydrocarbon feeds if the ratio between macropore volume and specific length and/or the total pore volume is outside these ranges.

Preferably, the ratio between macropore volume and specific length is above 0.20 ml/(g*mm), more preferably above 0.30 ml/(g*mm), and even more preferably above 0.40 ml/(g*mm), as well as preferably below 0.80 ml/(g*mm). It is further preferred that the catalyst has a total pore volume of at least 0.23 ml/g and most preferably of at least 0.25 ml/g.

Preferably, the catalyst particles have a specific length of at least 0.10 mm, more preferably of at least 0.16 mm, and most preferably of at least 0.20 mm. The upper limit of the specific length preferably lies at 2.0 mm, more preferably at 1.0 mm, and most preferably at 0.6 mm.

The particles of the catalyst can have many different shapes, including spheres, cylinders, rings, and symmetric or asymmetric polylobes, for instance tri- and quadrulobes. Preferably, the catalyst particles have an average particle diameter of at least 0.5 mm, more preferably of at least 0.8 mm, and most preferably of at least 1.0 mm. The upper limit of the average particle diameter preferably lies at 10.0 mm, more preferably at 5.0 mm, even more preferably at 3.0 mm.

Preferably, the macropore volume ranges from 0.05 to 0.30 ml/g, more preferably from 0.08 to 0.30 ml/g, and even more preferably from 0.08 to 0.25 ml/g.

The catalyst comprises a zeolite. Examples of zeolites contained in the catalyst are Y-zeolites, including H-Y-zeolites and USY-zeolites, zeolite beta, MCM-22, and MCM-36. Preferably, the zeolite is Y-zeolite with a unit cell size in the range of 24.34 - 24.72 angstroms. More preferably, the zeolite is a Y-zeolite with a unit cell size in the range of 24.40-24.61 angstroms and a silica:alumina molar ratio in the range 7-18 and most preferably the zeolite is a Y-zeolite with a unit cell size in the range of 24.24-24.58 angstroms and a silica:alumina molar ratio in the range 7.85-13.75.

As stated above, the catalyst comprises a hydrogenation function. A suitable hydrogenation function, e.g., comprises a Group VIII noble metal. The Group VIII noble metal preferably is contained in the catalyst in an amount of 0.01 - 2 wt%, and more preferably 0.1 - 1 wt%, calculated as metal and based on the weight of the zeolite. Preferably, the Group VIII noble metal comprises palladium and/or platinum.

Preferably, the catalyst additionally comprises a matrix material. Examples of suitable matrix materials are alumina, silica, titania, zirconia, clays, and mixtures thereof. Matrix materials comprising alumina are generally preferred.

Preferably, the catalyst comprises 2-98 wt% of the zeolite and 98-2 wt% of the matrix material, based on the total weight of the zeolite and matrix material present in the catalyst. More preferably, the catalyst comprises 10 - 90 wt% of the zeolite and 90 - 10 wt% of the matrix material, based on the total weight of the zeolite and matrix material contained in the catalyst. Even more preferably, the catalyst comprises 20 - 80 wt% of the zeolite zeolite and 80 - 20 wt% of the matrix material, most preferably 50 - 80 wt% of the zeolite and 20 - 50 wt% of the matrix material, based on the total weight of the zeolite and matrix material contained in the catalyst.

Preferably, the catalyst consists essentially of a hydrogenation function, a zeolite, and, optionally, a matrix material. More preferably, the catalyst consists essentially of a zeolite, a Group VIII noble metal, and a matrix material. It is further preferred that the catalyst is essentially free of rare earth metals and/or Group VIII non-noble metals. Thus, most preferably, the catalyst of the invention consists essentially of a Group VIII noble metal. compound, a zeolite, and a matrix, wherein

(i) the zeolite consists essentially of oxidic compounds (oxides and hydroxides) of a Group III element, such as aluminium, and/or of a Group IV element, such as silicon, and, optionally, oxidic compounds of a Group I element, such as sodium, and/or of a Group II element, such as calcium, and/or of a Group V element, such as phosphorus, and, optionally, additionally ammonium and/or water, and

(ii) the matrix is selected from the group of oxidic compounds of silicon, aluminum, titanium, zirconium, Group II metals or mixtures thereof.

The catalyst can be prepared by processes common to the industry. A typical process comprises the successive steps of

(i) shaping, e.g., extruding zeolite, optionally after mixing it with a matrix material, to form particles,
(ii) calcining the resulting particles, and
(iii) incorporating the hydrogenation function into the calcined particles by, e.g., impregnating the particles with a solution of a hydrogenation metal component and/or by (competitive) ion exchange.

Alternatively, the catalyst can, e.g., be prepared by a process comprising the successive steps of

(i) incorporating the hydrogenation function into the zeolite or into a mixture of the zeolite and the matrix material,
(ii) shaping, e.g., extruding the resulting material to form particles, and
(iii) calcining the resulting particles.

The catalyst is particularly suitable for the alkylation of saturated hydrocarbons. The invention therefore pertains to the use of the catalyst of the invention in the alkylation of these feedstocks. As stated above, this comprises the reaction of a saturated hydrocarbon with an olefin or olefin precursor in the presence of the catalyst of the invention to give highly branched saturated hydrocarbons with a higher molecular weight. Preferably, the hydrocarbon is a branched saturated hydrocarbon such as an isoalkane having 4 - 10 carbon atoms. Examples are isobutane, isopentane, isohexane or mixtures thereof, with isobutane being most preferred. The olefins to be used in the alkylation process generally have 2 - 10 carbon atoms, preferably 2 - 6 carbon atoms, still more preferably 3 - 5 carbon atoms, and most preferably 4 carbon atoms. Most preferably, the alkylation process consists of the alkylation of isobutane with butenes.

As will be evident to the skilled person, the alkylation process can be applied in any suitable form, including fluidized bed processes, slurry processes, and fixed bed processes. The process may be carried out in a number of beds and/or reactors, each with separate olefin addition. In such a case, the process of the invention may be carried out in each separate bed or reactor.

Suitable process conditions are known to the skilled person. Preferably, an alkylation process as disclosed in WO 9823560 is applied. The process conditions applied in this process are summarized in the following Table:

|  | Temperature range [°C] | pressure range [bar] | molar ratio of saturated hydrocarbon to olefin |
|---|---|---|---|
| preferred | -40 - 250 | 1-100 | 5:1 - 5000:1 |
| more preferred | 0-150 | 10 - 40 | 50:1 - 1000:1 |
| most preferred | 60 - 95 | 15 - 30 | 150:1 - 750:1 |

Preferably, a regeneration technique as described in WO 9823560 is applied during the alkylation process. More in particular, during the alkylation process the catalyst is preferably subjected intermittently to a regeneration step by being contacted with a feed containing an aliphatic compound and hydrogen, with said regeneration preferably being carried out at 90% or less, more preferably at 60% or less, even more preferably at 20% or less, and most preferably at 10% or less of the active cycle of the catalyst. The active cycle of the catalyst is defined as the time from the start of the feeding of the alkylation agent to the moment when, in comparison with the entrance of the catalyst-containing reactor section, 20% of the alkylation agent leaves the catalyst-containing reactor section without being converted, not counting isomerisation inside the molecule. Optionally, in this process, the catalyst can be subjected periodically to a high-temperature regeneration with hydrogen in the gas phase. This high-temperature regeneration is preferably carried out at a temperature of at least 150°C, more preferably at 175° - 600°C, and most preferably at 200° - 400°C. For details of this regeneration procedure, reference is made to WO 9823560, and in particular to page 4, lines 5 - 19 and page 9, line 13 through page 13, line 2.

The use of the catalyst in the above alkylation process results in a high olefin conversion (amount of olefin in the feed that is converted in the reaction), a high C5+ alkylate yield (weight amount of C5+ alkylate produced divided by the overall weight of olefin consumed) and a high octane number, while the amount of undesired C9+ by-products can be restricted and the catalyst's stability can thus be improved. For details in respect of these parameters, reference is made to WO 9823560.

The following characterization method was applied in the present invention:

The macropore volume as well as the total pore volume were determined via mercury intrusion on the basis of the Washbum equation

$$D = \frac{-4\gamma \cos\theta}{p}$$

with D being the pore diameter, p being the pressure applied during the measurement, $\gamma$ being the surface tension, taken to be 480 dynes/cm, and $\theta$ being the contact angle, taken to be 140°. In the present measurement, the pressure was varied over such a range that the measurement covered pores with a diameter in the range of 3.6 - 8000 nm.

The present invention will be further illustrated by way of the following examples:

General test procedure

A fixed-bed recycle reactor as described in WO 9823560 having a diameter of 2 cm was filled with a 1:1 volume/volume mixture of 38.6 grams of catalyst extrudates and carborundum particles (60 mesh). At the centre of the reactor tube a thermocouple of 6 mm in diameter was arranged. The reactor was flushed with nitrogen for 30 minutes (100 Nl/hour). Next, the system was tested for leakages at elevated pressure, after which the pressure was raised to 21 bar and the nitrogen replaced by hydrogen (100 Nl/hour). The reactor temperature was then raised to 200°C. at a rate of 1°C/min. After 1 hour at 200°C the temperature was raised to 400°C at a rate of 1°C/min. After 1 hour at 400°C the reactor temperature was lowered to the reaction temperature, which is given in the Examples below.

The hydrogen stream was stopped with the attaining of the reaction temperature. Isobutane was supplied to the reactor at a rate of about 4,000 grams/hour. About 95 - 98% of the isobutane was fed back to the reactor. About 2 - 5% was drained off for analysis. Such an amount of isobutane was supplied to the reactor to ensure a constant quantity of liquid in the system. When the system had stabilized, such an amount of cis-2-butene was added to it as to give a cis-2-butene-WHSV as given in the examples below (calculated on zeolite weight in the catalyst sample). The overall rate of flow of liquid in the system was maintained at about 4,000 g/h. The weight ratio of isobutane to cis-2-butene in the reactor supply (without considering the unreacted material fed back to the reactor) is given in the Examples below. The pressure in the reactor amounted to 21 bar.

Each time after 1 hour of reaction, the catalyst was regenerated by being washed with isobutane for 5 minutes, followed by 50 minutes of regeneration through being contacted with a solution of 1 mol% of H2 in isobutane, and then being washed with isobutane for another 5 minutes (total washing and regeneration time 1 hour). After this washing step, alkylation was started again. The process conditions during the washing steps and the regeneration step were the same as the process conditions during the reaction step.

Unless specified otherwise, the catalytic performance was measured after a steady state was reached. The performance was characterized by the olefin conversion, the research octane number (RON), the C5+ alkylate yield, and the weight percentage of undesired C9+ by-products (excl. 2,2,5-trimethylhexane), calculated on C5+ alkylate. The RON was determined as described on pages 13 and 14 of WO 9823560, the only exception being that the RON contribution of total C9+ (excl. 2,2,5-trimethylhexane) was estimated to be 84 instead of 90. The C5+ alkylate yield is defined as the weight amount of C5+ alkylate produced divided by the overall weight of olefin consumed.

Example 1:

A catalyst of the invention was tested according to the above-described test procedure. The weight ratio of isobutane to cis-2-butene in the reactor supply (without considering the unreacted material fed back to the reactor) was 20. The reaction temperature was 70°C. The cis-2-butene-WHSV was 0.21 h-1. The catalyst had the following properties:

| Catalyst composition: | |
| --- | --- |
| Solid acid: | USY-zeolite |
| Solid acid amount: | 70 wt% (based on the total weight of solid acid and matrix) |
| Hydrogenation metal: | platinum |
| Hydrogenation metal amount: | 0.34 wt% |
| Matrix: | alumina |
| Matrix amount: | 30 wt% (based on the total weight of solid acid and matrix) |
| Catalyst shape: | cylindrical extrudates |
| Pore/particle characteristics: | |
| Macropore volume: | 0.17 ml/g |
| Specific length: | 0.22 mm (average diameter: 1.0 mm, average length: 4 mm) |
| Macropore volume / specific length | 0.77 ml/(g*mm) |
| Total pore volume: | 0.36 ml/g |

The catalytic performance is given in the Table below.

Example 2:

A catalyst with the same composition and shape as the catalyst of Example 1 was tested. The catalyst had a ratio between macropore volume and specific length of 0.64 ml/(g*mm) (macropore volume: 0.14 ml/g, specific length: 0.22 mm (average diameter: 1.0 mm, average length: 4 mm)). Its total pore volume was 0.35 ml/g. The weight ratio of isobutane to cis-2-butene in the reactor supply (without considering the unreacted material fed back to the reactor) was 19. The reaction temperature was 70°C. The cis-2-butene-WHSV was 0.21 h-1. The further test conditions were as described in Example 1. The catalytic performance is given in the Table below.

Example 3:

A catalyst with the same composition and shape as the catalyst of Example 1 was tested. It had a ratio between macropore volume and specific length of 0.41 ml/(g*mm) (macropore volume: 0.09 ml/g, specific length: 0.22 mm (average diameter: 1.0 mm, average length: 4 mm)). Its total pore volume was 0.27 ml/g. The weight ratio of isobutane to cis-2-butene in the reactor supply (without considering the unreacted material fed back to the reactor) was 19. The reaction temperature was 70°C. The cis-2-butene-WHSV was 0.21 h-1. The further test conditions were as described in Example 1. The catalytic performance is given in the Table below.

Example 4

A catalyst with the same composition and shape as that of Example 1 was tested. It had a ratio between macropore volume and specific length of 0.49 ml/(g*mm) (macropore volume: 0.17 ml/g, specific length: 0.35 mm (average diameter: 1.7 mm, average length: 4 mm)). Its total pore volume was 0.38 ml/g. The weight ratio of isobutane to cis-2-butene in the reactor supply (without considering the unreacted material fed back to the reactor) was 26. The reaction temperature was 80°C. The cis-2-butene-WHSV was 0.19 h-1. The further test conditions were as described in Example 1. The catalytic performance is given in the Table below.

Comparative Example A

A catalyst with the same composition and shape as the catalyst of Example 1 was tested. It had a ratio between macropore volume and specific length of 0.95 ml/(g*mm) (macropore volume: 0.21 ml/g, specific length: 0.22 mm (average diameter: 1.0 mm, average length: 4 mm)), which lies outside the claimed range. Its total pore volume was 0.50 ml/g. The weight ratio of isobutane to cis-2-butene in the reactor supply (without considering the unreacted material fed back to the reactor) was 30. The reaction temperature was 70°C. The cis-2-butene-WHSV was 0.21 h-1. The further test conditions were as described in Example 1. The catalytic performance is given in the Table below.

Comparative Example B

A catalyst with the same composition and shape as the catalyst of Example 1 was tested. It had a ratio between macropore volume and specific length of 0.18 ml/(g*mm) (macropore volume: 0.04 ml/g, specific length: 0.22 mm (average diameter: 1.0 mm, average length: 4 mm)). Its total pore volume was 0.19 ml/g, which lies outside the claimed range. The weight ratio of isobutane to cis-2-butene in the reactor supply (without considering the unreacted material fed back to the reactor) was 27. The reaction temperature was 80°C. The cis-2-butene-WHSV was 0.19 h-1. The further test conditions were as described in Example 1. As a steady state could not be reached, the catalytic performance was measured after 60 hours. The results are given in the Table below.

Discussion:

The catalytic performance of the catalysts of the above Examples is summarized in the Table below:

| | Ex. 1 | Ex. 2 | Ex. 3 | Ex. 4 | Com. Ex. A | Com. Ex. B |
|---|---|---|---|---|---|---|
| Total pore volume [ml/g] | 0.36 | 0.35 | 0.27 | 0.38 | 0.50 | 0.19 |
| Macropore volume/ specific length [ml/(g*mm)] | 0.77 | 0.64 | 0.41 | 0.49 | 0.95 | 0.18 |
| Olefin conversion [%] | 100 | 99.9 | 100 | 99.7 | 99.5 | 98.4 |
| wt% C9+ (excl. 2,2,5-trimethyl-hexane), calc. on C5+ alkylate | 5.3 | 6.4 | 5.0 | 6.1 | 13 | 10 |
| RON (research octane number) | 95.7 | 96.0 | 96.1 | 95.7 | 95.6 | 95.0 |
| C5+ alkylate yield [%] | 205 | 207 | 206 | 202 | 185 | 183 |

The performance of the catalysts of Comparative Examples A and B is significantly worse than that in Examples 1 - 4. More in particular, the C5+ alkylate yield is significantly lower than the corresponding yields of the catalysts according to the invention, whereas the weight percentage of undesired C9+ lies significantly above the corresponding values of the catalysts of Examples 1 - 4.
It is noted that this poor performance of the catalysts of Comparative Examples A and B is observed despite the fact that they were tested at conditions that should lead to a better catalytic performance than the conditions under which the catalysts of Examples 1 - 4 were tested. More in particular, the weight ratio between isobutane and cis-2-butene in Comparative Examples A and B is higher than in Examples 1 - 4. A higher weight ratio implies a lower amount of olefin in the reactor and thus a lower risk of excess olefin being able to react with the formed alkylate resulting in undesired C9+ products.

## Claims

1. Use of a catalyst for the alkylation of hydrocarbons, which catalyst comprises a hydrogenation function and a zeolite, wherein the ratio between (i) the volume in catalyst pores with a diameter in the range of 40 - 8000 nm and (ii) the specific length of the catalyst particles is in the range of 0.01 - 0.90 ml/(g*mm) and wherein the catalyst has a total pore volume of at least 0.20 ml/g and the volume in the catalyst pores with a diameter in the range of 40-8000 nm is below 0.30 ml/g.

2. Use according to claim 1, **characterized in that** the hydrocarbons are saturated hydrocarbons.

3. Use according to claim 1 or 2, in which the ratio between (i) the volume in catalyst pores with a diameter in the range of 40 - 8000 nm and (ii) the specific length of the catalyst particles is at least 0.20 ml/(g*mm).

4. Use according to any one of the preceding claims wherein the catalyst has a total pore volume of at least 0.23 ml/g.

5. Use according to any one of the preceding claims wherein the hydrogenation function consists essentially of a Group VIII noble metal.

6. Use according to any one of the preceding claims wherein the zeolite is a Y-zeolite with a unit cell size in the range of 24.34 - 24.72 angstroms.

7. Use according to claim 6 wherein the Y zeolite has a unit cell size in the range of 24.40-24.61 angstroms.

8. Use according to claim 7 wherein the Y zeolite has a unit cell size in the range of 24.45-24.58 angstroms.

9. Use according to any one of the preceding claims wherein the catalyst additionally comprises a matrix material.

10. Use according to claim 9 wherein the matrix material comprises alumina.

11. Use according to any one of the preceding claims wherein the catalyst is free of rare earth metals and Group VIII non-noble metals.

## Patentansprüche

1. Verwendung eines Katalysators für die Alkylierung von Kohlenwasserstoffen, wobei der Katalysator eine Hydrierungsfunktion und einen Zeolithen umfasst, wobei das Verhältnis zwischen (i) dem Volumen in den Katalysatorporen mit einem Durchmesser im Bereich von 40 - 8000 nm und (ii) der spezifischen Länge der Katalysatorteilchen im Bereich von 0,01 ml/(g*mm) und 0,90 ml/(g*mm) liegt, und wobei der Katalysator ein Gesamtporenvolumen von wenigstens 0,20 ml/g hat und das Volumen in den Katalysatorporen mit einem Durchmesser im Bereich von 40 - 8000 nm kleiner als 0,30 ml/g ist.

2. Verwendung gemäß Anspruch 1, **dadurch gekennzeichnet, dass** die Kohlenwasserstoffe gesättigte Kohlenwasserstoffe sind.

3. Verwendung gemäß den Ansprüchen 1 oder 2, wobei das Verhältnis zwischen (i) dem Volumen in den Katalysatorporen mit einem Durchmesser im Bereich von 40 - 8000 nm und (ii) der spezifischen Länge der Katalysatorteilchen wenigstens 0,20 ml/(g*mm) ist.

4. Verwendung gemäß irgendeinem der vorhergehenden Ansprüche, wobei der Katalysator ein Gesamtporenvolumen von wenigstens 0,23 ml/g hat.

5. Verwendung gemäß irgendeinem der vorhergehenden Ansprüche, wobei die Hydrierungsfunktion im Wesentlichen aus einem Edelmetall der Gruppe VIII besteht.

6. Verwendung gemäß irgendeinem der vorhergehenden Ansprüche, wobei der Zeolith ein Y-Zeolith mit einer Einheitszellengröße im Bereich von 24,34 Å bis 24,72 Å ist.

7. Verwendung gemäß Anspruch 6, wobei der Y-Zeolith eine Einheitszellengröße im Bereich von 24,40 Å bis 24,61 Å hat.

8. Verwendung gemäß Anspruch 7, wobei der Y-Zeolith eine Einheitszellengröße im Bereich von 24,45 Å bis 24,58 Å hat.

9. Verwendung gemäß irgendeinem der vorhergehenden Ansprüche, wobei der Katalysator zusätzlich ein Matrixmaterial umfasst.

10. Verwendung gemäß Anspruch 9, wobei das Matrixmaterial Aluminiumoxid umfasst.

11. Verwendung gemäß irgendeinem der vorhergehenden Ansprüche, wobei der Katalysator frei von Seltenerdmetallen und Nicht-Edelmetallen der Gruppe VIII ist.


**Revendications**

1. Utilisation d'un catalyseur pour l'alkylation d'hydrocarbures, lequel catalyseur comprend une fonction d'hydrogénation et une zéolite, dans laquelle le rapport entre (i) le volume représenté par les pores du catalyseur possédant un diamètre entre 40 et 8000 nm et (ii) la longueur spécifique des particules du catalyseur, est de l'ordre de 0,01 à 0, 90 ml/(g*mm) et dans laquelle le catalyseur présente un volume de pores total d'au moins 0,20 ml/g et le volume représenté par les pores du catalyseur possédant un diamètre entre 40 et 8000 nm est inférieur à 0,30 ml/g.

2. Utilisation selon la revendication 1, **caractérisé en ce que** les hydrocarbures sont des hydrocarbures saturés.

3. Utilisation selon la revendication 1 ou 2, dans laquelle le rapport entre (i) le volume représenté par les pores du catalyseur possédant un diamètre entre 40 et 8000 nm et (ii) la longueur spécifique des particules du catalyseur est d'au moins 0,20 ml/(g*mm).

4. Utilisation selon l'une quelconque des revendications précédentes dans laquelle le catalyseur présente un volume total de pores d'au moins 0,23 ml/g.

5. Utilisation selon l'une quelconque des revendications précédentes dans laquelle la fonction d'hydrogénation comprend essentiellement un métal noble du groupe VIII.

6. Utilisation selon l'une quelconque des revendications précédentes, dans laquelle la zéolite est une zéolite Y présentant une taille d'unité cellulaire de l'ordre de 24,34 à 24,72 angstroms.

7. Utilisation selon la revendication 6, dans laquelle la zéolite Y présente une taille d'unité cellulaire de l'ordre de 24,40 à 24,61 angströms.

8. Utilisation selon la revendication 7, dans laquelle la zéolite Y présente une taille d'unité cellulaire de l'ordre de 24,45 à 24,58 angstroms.

9. Utilisation selon l'une quelconque des revendications précédentes, dans laquelle le catalyseur comprend en outre un matériau de matrice.

10. Utilisation selon la revendication 9, dans laquelle le matériau de matrice comprend de l'alumine.

11. Utilisation selon l'une quelconque des revendications précédentes, dans laquelle le catalyseur est exempt de métaux terreux rares et de métaux non nobles du groupe VIII.